# EUROPEAN PATENT APPLICATION

(11) **EP 3 505 204 A1**
(43) Date of publication of application: **03.07.2019**
(21) Application number: 17843594.7
(22) Date of filing: 22.08.2017
(51) Int. Cl.: A61M 5/303

(54) **NEEDLELESS INJECTOR**

(30) Priority: 23.08.2016 JP 2016162870
(71) Applicant: Daicel Corporation, Osaka-shi, Osaka 530-0011 (JP)
(72) Inventor: YAMAMOTO, Yuzo, Tatsuno-shi, Hyogo 671-1681 (JP)
(74) Representative: Jones, Nicholas Andrew
(86) International application number: PCT/JP2017/029981
(87) International publication number: WO 2018/038116

(57) **Abstract**

An injector including main body, a syringe portion, a piston portion, an igniting device, and a pressing member, the pressing member is formed so as to have a part displaceable toward the piston portion while the part being kept from returning to the igniting device side upon receiving the ejection energy from the igniting device, and pressing the piston portion toward the plunger due to displacement of the part. In addition, while the piston portion is pressed by the pressing member, the elastically deformable part is partly or entirely recovered from the elastic deformation from a most compressed state by the pressing force from the pressing member so as to push the output portion toward the plunger. In this way, the injector achieves efficient ejection of the injection objective substance in order to deliver the injection objective substance to an injection target area while the injector is designed to be as compact as possible.

## Description

### [Technical Field]

The present invention relates to a needleless injector for injecting an injection objective substance into an injection target area of a subject.

### [Background Art]

Regardless of the presence or absence of an injection needle, an injection solution is ejected by pressurizing the injection solution by an injector. In a case of a needleless injector, which makes an injection without using an injection needle, gunpowder is sometimes used as a pressurizing source (see for example PTL 1). The needleless injector disclosed in PTL 1 includes a detonator and an explosive charge. A firing hammer sticks the detonator for igniting the detonator, and the thermal energy generated by the ignition is transmitted to the explosive charge. Then, the explosive charge burns to pressurize an injection solution. As such an explosive charge, a single kind of nitrocellulose-based gunpowder is used.

In a needleless injector with no injection needle, an amount of pressurized injection solution itself penetrates the skin of a subject and is delivered into the subject. For example, PTL 2 discloses a technique for adjusting a force upon penetrating the skin in a needleless injector. According to the technique, a piston structure for pressurizing an injection solution includes a ram and a sleeve, which are connected via a spring. Before injection, a prescribed clearance D2 is set between the ram and the sleeve, and the spring is compressed by the clearance D2 upon injection, so that the pressure to be applied on the injection solution upon injection, in other words, a skin penetrating force is adjusted. Therefore, the skin penetrating force can be adjusted by adjusting the clearance D2.

### [Citation List]

### [Patent Literature]

[PTL 1] Japanese Translation of PCT Application No. 2004-532049
[PTL 2] U. S. Patent No. 5722953 (Specification)

### [Summary of Invention]

### [Technical Problem]

In order to pressurize an injection solution in a needleless injector and push the solution to penetrate the skin, which is an injection target, as in the conventional examples, energy necessary for the pressurization should be sufficiently transmitted to the injection solution to be ejected. In the conventional examples mentioned above, a spring is used in a piston structure to adjust force used for pushing an injection solution to penetrate the skin. In this case, the prescribed clearance D2 between the ram and the sleeve is required in a state prior to injection. As a result, the length of the needleless injector inevitably increases by the clearance D2, which may not be necessarily desirable in terms of user's convenience.

In view of the issues as mentioned above, it is an object of the present invention to provide a needleless injector for ejecting an injection objective substance capable of achieving efficient ejection of the injection objective substance in order to deliver the injection objective substance to an injection target area while the injector is designed to be as compact as possible.

### [Solution to Problem]

In order to achieve the object, the present invention adopts, as a structure of a needleless injector, a structure that transmits ejection energy supplied by combustion of gunpowder in an igniting device to a piston portion through a pressing member, wherein a part of the ejection energy is accumulated in the piston portion in the form of elastic energy while at least a part of the accumulated energy is released in the sliding process of the piston portion. Since the piston portion is pressed by the pressing member upon release of the elastic energy, the released elastic energy can optimally be transmitted to the injection objective substance through a plunger positioned ahead of the piston portion.

Specifically, the present invention relates to a needleless injector for injecting an injection objective substance to an injection target area without using an injection needle, the needleless injector including: an injector main body having a through hole formed in an axial direction thereof; a piston portion provided slidably in the through hole; a syringe portion provided on a tip end side of the injector main body, the syringe portion including a storing chamber capable of storing the injection objective substance, a plunger to pressurize the injection objective substance in the storing chamber as the piston portion slides; a nozzle portion that includes a flow passage for allowing the injection objective substance in the storing chamber pressurized by the plunger to be flown therethrough and ejects the injection objective substance from an ejection outlet formed at a tip end of the flow passage; an igniting device provided at a base end side of the injector main body to burn gunpowder, the igniting device supplying ejection energy by combustion of the gunpowder in the igniting device; and a pressing member provided between the piston portion and the igniting device, the pressing member being formed so as to have a part displaceable toward the piston portion while the part being kept from returning to the igniting device side upon receiving the ejection energy from the igniting device, and pressing the piston portion toward the plunger due to displacement of the part. The piston portion has an elastically deformable part which elastically deforms in a sliding direction of the piston portion upon receiving pressing force from the pressing member, and an output portion provided in contact with the plunger and connected to the elastically deformable part, and in the sliding process of the piston portion, while the piston portion is pressed by the pressing member, the elastically deformable part is partly or entirely recovered from the elastic deformation from a most compressed state by the pressing force from the pressing member so as to push the output portion toward the plunger.

In the needleless injector according to the present invention, the ejection energy generated by the combustion of the gunpowder in the igniting device causes a part of the pressing member to be displaced toward the side of the piston portion, and the piston portion is pressed to the plunger side due to the displacement, so that the piston portion is allowed to slide in the through hole. The sliding of the piston portion pressurizes the injection objective substance stored in the sorting chamber of the syringe portion through the plunger, so that the injection objective substance is ejected externally from the injector through the ejection outlet. Note that if the displacement of the part of the pressing member is caused by the combustion of the gunpowder, a configuration in which the ejection energy may directly act on the piston portion through the pressing member, a configuration in which the ejection energy may be once propagated to another material such as gas, liquid, and solid and then may eventually be made to act indirectly upon the piston portion through the pressing member, or any other configurations may be used as appropriate.

The injection objective substance contains a component expected to have efficacy in the injection target area, and if the injection objective substance can be ejected by the ejection energy, the injection objective substance may be stored in any form in the injector or the injection objective substance may be in any physical form such as fluid such as liquid and gel, powder, and solid in a granular form. The injection objective substance contains a component to be delivered into an injection target area of a living body as a subject, and the component may be dissolved in the injection objective substance or may be simply mixed without dissolution. Examples of the component to be delivered include a vaccine for enhancing an antibody, protein for a cosmetic purpose, and cultured cells for hair regeneration, and the injection objective substance is formed by including any of these components in a fluid form such as liquid and gel so that the component can be ejected.

Here, in the needleless injector according to the present invention, the igniting device for combusting gunpowder may be any device in which the gunpowder stored therein is ignited by operation of the igniting device and a combustion product of the gunpowder is generated or a known gas generation agent (such as single base smokeless gunpowder) may also be combusted by the ignition of the gunpowder, so that the combustion products of the gunpowder and the gas generation agent are generated. In the needless injector according to the present invention, the specific structure of the igniting device is not limited. When the gunpowder is combusted in the igniting device, the combustion product is diffused in the space of the injector main body, and the ejection energy is transmitted to the piston portion generally through pressure, heat, etc., and the energy serves as a motive power source for ejection of the injection objective substance as described above.

Here, the pressing member may be in any form if the pressing member is formed to have a part displaceable toward the piston portion while the part is kept from returning to the igniting device side upon receiving the ejection energy from the igniting device. For example, the pressing member may plastically deform toward the piston portion upon receiving the ejection energy from the igniting device, so that a part of the pressing member is displaced toward the piston portion and presses the piston portion. By using such plastic deformation as described above, the piston portion can be pressed efficiently and the piston portion to pressurize the injection objective substance through the plunger can be restrained from being returned to the igniting device side by reaction force received from the plunger. Alternatively, the pressing member may be configured like a ratchet, so that displacement only in a single direction is allowed and the piston portion may be pressed by taking advantage of the displacement. Also in this case, the piston portion can be restrained from being returned to the igniting device side during pressurization of the injection objective substance. Since the pressing member is displaced only in a single direction toward the piston portion, the piston portion may be pressed efficiently by the pressing member.

The piston portion which receives pressing force from the pressing member has the elastically deformable part and the output portion. The elastically deformable part elastically deforms by the received pressing force, so that a part of the ejection energy can be accumulated as elastic energy. The output portion in contact with the plunger can directly transmit the pressing force received by the piston portion to the plunger and can also transmit the elastic energy accumulated by the elastically deformable part to the plunger.

Here, in the process in which the piston portion is pressed by the pressing member to slide in the through hole, the elastically deformable part elastically deforms by the received pressing force, and the ejection energy is partly accumulated as elastic energy. Meanwhile, the rest of the ejection energy is basically transmitted to the plunger side. In the sliding process of the piston portion, the piston portion is pressed by the pressing member, and the pressing member is displaced toward the piston side while being kept from returning to the igniting device side as described above, so that the elastically deformable part has its elastic deformation partly or entirely recovered from a most compressed state, and the output portion is pushed out toward the plunger, which releases the elastic energy toward the plunger. As a result, the elastic energy is transmitted to the plunger in addition to the rest of the ejection energy which is directly transmitted. The release of the elastic energy is delayed from the direct transmission of the ejection energy, so that the injection objective substance is pressurized in the later stage in the sliding process of the piston portion. As a result, the combustion of the gunpowder in the igniting device allows the piston portion to pressurize the injection objective substance for a relatively long period of time through the plunger. This leads to efficient ejection of the injection objective substance, so thin the igniting device as well as the needleless injector may be reduced in size.

In the needleless injector, the pressing member may be formed to separate the space in the injector main body into a first space in which the igniting device is provided and a second space in which the piston portion is provided and to seal a combustion product generated by the igniting device in the first space. According to this configuration, the combustion product is sealed within the first space and does not enter the second space. Therefore, any undesirable effect of the combustion product on the injection objective substance can be reduced.

In the needleless injector, the pressing member may be made of a metal plate member or a metal film member. The pressing member made of a metal has a certain level of resistance against the combustion of the gunpowder in the igniting device, and the member in the plate or film shape can be relatively easily displaced toward the piston portion upon receiving the ejection energy, so that efficient ejection of the injection objective substance is achieved.

Here, in the needleless injector, a reinforcement member which reinforces the pressing member in a thickness-wise direction may be provided in a prescribed location of the pressing member in contact with the piston portion. The prescribed location is at a part of the pressing member, which comes in contact with the piston portion, and therefore relatively large stress is considered to be applied thereon during displacement in response to the ejection energy. Therefore, in order to surely press the piston portion or maintain sealing force if the combustion product of the gunpowder is sealed in the first space, it is undesirable that the pressing member is damaged while pressing the piston portion. Therefore, damages to the pressing member may be avoided by providing the reinforcement member in the prescribed location.

### [Advantageous Effects of Invention]

A needleless injector for ejecting an injection objective substance according to the present invention can achieve efficient ejection of the injection objective substance in order to deliver the injection objective substance to an injection target area while the injector is designed to be as compact as possible.

### [Brief Description of Drawings]

Fig. 1 is a view of a general structure of an injector according to a first embodiment of the present invention.
Fig. 2 is a view showing details of the piston of the injector shown in Fig. 1.
Fig. 3 is a view of a general structure of an initiator (an igniting device) mounted to the injector shown in Fig. 1.
Fig. 4 is a view showing in comparison the injector in Fig. 1 in a state before gunpowder is combusted in the initiator, a state in the process of ejecting an injection solution, and a state at the completion of ejection of the injection solution.
Fig. 5 is a view of a general structure of an injector according to a second embodiment of the present invention.

### [Description of Embodiments]

Now, an injector according to an embodiment of the present invention will be described in conjunction with the drawings by referring to a needleless injector 1 having no an injection needle (hereinafter simply as the "injector 1") as an example. Note that the configuration in the following embodiment is illustrated by way of example and not intended to limit the present invention.

### <First Embodiment>

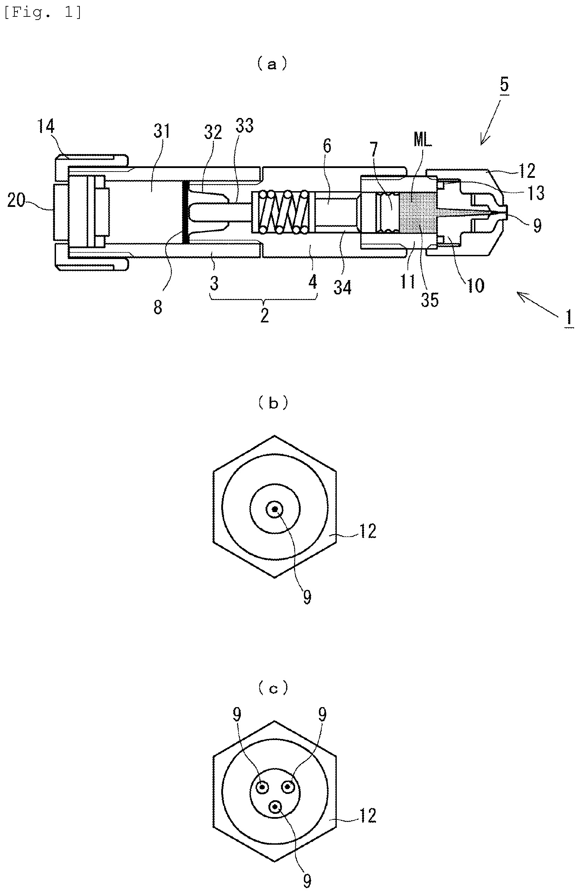

Fig. 1(a) is a sectional view of the injector 1, and Fig. 1(b) is a view of the injector 1 from the side of a nozzle portion 9 which ejects an injection solution. Note that in the following description, an injection objective substance to be injected to an injection target area of a subject by the injector 1 will generically be referred to as the "injection solution." However, this is not intended to limit the content or form of the substance to be injected. A component to be delivered to a skin structure may or may not be dissolved in the injection objective substance or the injection objective substance may be in any of various forms such as liquid, gel, and powder states if the injection solution can be pressurized and thus ejected from the nozzle portion 9 to the skin structure.

Here, the injector 1 has an injector main body 2 including a first housing and a second housing 4, and a syringe portion 5 is provided at the tip end side of the injector main body 2 (on the end side opposite to the end of the second housing 4 connected to the first housing 3). The first housing and the second housing 4 are screwed together. Here, a combustion chamber 31 as an internal space extending in the axial direction is formed in the first housing 3, and a through hole 34 also as an internal space extending along the axial direction is formed in the second housing 4. The combustion chamber 31 and the through hole 34 are separated by a pressing member 8 but formed continuously as internal spaces in the injector main body 2. The pressing member 8 is made of a plate-shaped or film shaped metal member.

The syringe portion 5 provided on the tip end side of the injector main body 2 has a syringe portion main body 11 having therein a storing chamber 35 which stores an injection solution ML, the nozzle portion 9 having a flow passage in which the injection solution flows, and a nozzle main body 10 provided with the nozzle portion 9. The nozzle main body 10 is attached to the syringe portion main body 11 by a nozzle holder 12 with a gasket 13 therebetween. The syringe portion main body 11 is screwed to the end of the second housing 4 of the injector main body 2, and in the attached state, the through hole 34 in the second housing 4 and the storing chamber 35 in the syringe portion main body 11 form a continuous space. Note that in the attached state, the injection solution ML is stored liquid-tightly in the storing chamber 35 by a plunger 7, and the plunger 7 is exposed to the side of the through hole 34. Here, the plunger 7 is provided slidably in the storing chamber 35, and as the plunger 7 slides to pressurize the injection solution ML, the injection solution is ejected from the nozzle portion 9. The plunger 7 is made of a rubber member having a thin coating of silicon oil on the surface so that the plunger 7 can slide smoothly in the storing chamber 35.

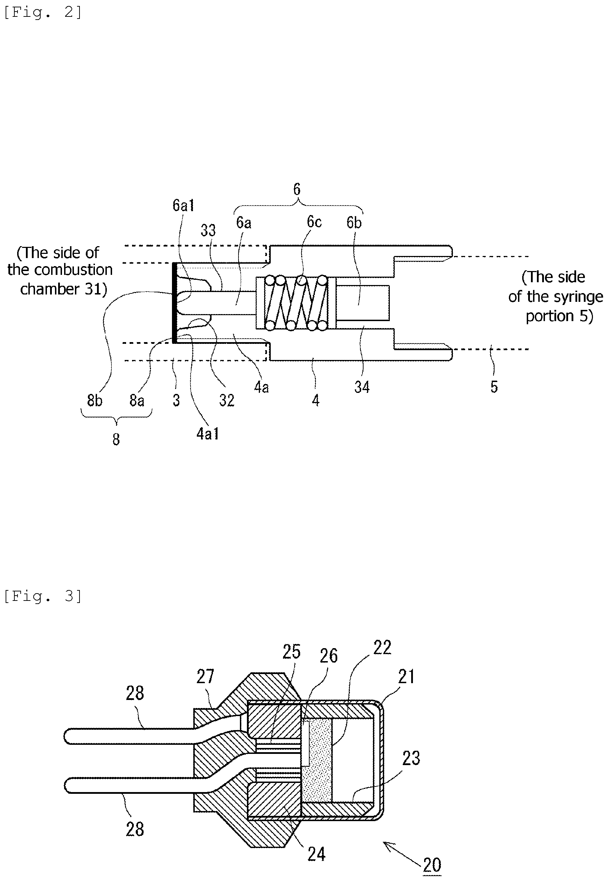

The metal piston portion 6 is provided in the through hole 34 in the second housing 4 of the injector main body 2, and the piston portion 6 is held slidably in the through hole 34. Here, Fig. 2 is a view for better understanding of the positional relation between the piston portion 6 and the second housing 4. The piston portion 6 is formed to extend substantially axially along the axis of the through hole 34 and has an end portion (hereinafter also referred to as a "first end portion") 6a on the side of the combustion chamber 31, an end portion (hereinafter also referred to as a "second end portion") 6b on the side of the syringe portion 5, i.e., an end portion in contact with the plunger 7 provided at the syringe portion 5, and a spring part 6c which connects the first end portion 6a and the second end portion 6b. The spring part 6c is provided to be elastically deformable along the axis of the through hole 34. Note that in the state shown in Fig. 1, i.e., in the state before gunpowder is combusted in an initiator 20 (hereinafter referred to as a "pre-ignition state") as an igniting device which will be described, the spring part 6c is in its natural length, and no elastic force acts on the first end portion 6a and the second end portion 6b from the spring part 6c.

Here, the first housing 3 (indicated by the dotted line in Fig. 2) and the second housing 4 are attached to each other to form the injector main body 2, and in the pre-ignition state before the gunpowder is combusted in the initiator 20 as the igniting device which will be described, the first end portion 6a passes through the through hole 33 provided at the fitting part 4a of the second housing 4 fitted in the combustion chamber 31 of the first housing 3, and the end face 6a1 of the first end portion 6a is provided at the fitting part 4a to substantially protrude to the side of the combustion chamber 31 from the bottom surface of a recess 32 continuous with the through hole 33. The through hole 33 connects the through hole 34 and the recess 32. Note that the end surface 6a1 of the first end portion 6a protrudes from the bottom surface of the recess 32 to be substantially flush with the end face 4a1 of the fitting part 4a.

A peripheral part 8a of the pressing member 8 is fixed at the end face 4a1 of the fitting part 4a, and in this state, the center part 8b of the pressing member 8 is in contact with or in close proximity with the first end portion 6a protruding from the bottom surface in the recess 32. Note that as for the fixing of the peripheral part 8a to the fitting part 4a, the peripheral part 8a is fixed to the fitting part 4a so that the combustion product of gunpowder generated in the initiator 20 is sealed to the side of the combustion chamber 31, or that the combustion product is prevented from moving from the combustion chamber 31 to the injector main body 2. In this way, ejection energy supplied from the initiator 20 is transmitted to the piston portion 6 form the pressing member 8. The transmission will be described in detail.

Here, an example of the initiator 20 will be described with reference to Fig. 3. The initiator 20 is an electric igniting device, and a cup 21 having its surface covered with an insulation cover defines a space for providing a gunpowder 22 in the cup 21. A metal header 24 is provided in the space, and a tubular charge holder 23 is provided on an upper surface thereof. The charge holder 23 holds the gunpowder 22. A bridge wire 26 which electrically connects one of conduction pins 28 and the metal header 24 is provided at the bottom of the gunpowder 22. Note that the two conduction pins 28 are fixed to the metal header 24 through an insulator 25 so that these conduction pins are insulated from each other when there is no voltage application. The opening of the cup 21 from which the two conduction pins 28 supported by the insulator 25 extend are protected while the insulation between the conduction pins 28 is well maintained by a resin collar 27.

In the initiator 20 having the above-described structure, when voltage is applied across the region between the two conduction pins 28 by an external power supply, current is passed through the bridge wire 26, which causes the gunpowder 22 to be combusted. At the time, a combustion product generated by the combustion of the gunpowder 22 is discharged from the opening of the charge holder 23. An initiator cap 14 has a flange-shaped section to be hooked at the outer surface of the initiator 20 and is screwed to the first housing 3. In this way, the initiator 20 is fixed to the first housing 3 by the initiator cap 14, so thin the initiator 20 itself can be prevented from being dropped from the injector main body 2 by pressure generated upon ignition in the initiator 20.

Here, examples of the gunpowder 22 used in the injector 1 preferably include gunpowder containing zirconium and potassium perchlorate (ZPP), gunpowder containing titanium hydroxide and potassium perchlorate (THPP), gunpowder containing titanium and potassium perchlorate (TiPP), gunpowder containing aluminum and potassium perchlorate (APP), gunpowder containing aluminum and bismuth oxide (ABO), gunpowder containing aluminum and molybdenum oxide (AMO), gunpowder containing aluminum and copper oxide (ACO), gunpowder containing aluminum and iron oxide (AFO), and gunpowder consisting of a combination of two or more of these kinds of gunpowder. These kinds of gunpowder generate high temperature high pressure plasma during combustion immediately after ignition but the generation pressure abruptly drops at normal temperatures at which the combustion product is condensed and does not contain a gas component. Note that gunpowder other than the above may be used.

Although no substance is provided in the combustion chamber 31 shown in Fig. 1, a gas generation agent which is combusted by the combustion product generated by the combustion of the gunpowder 22 and generates a gas may be provided in the combustion chamber 31. If such a gas generation agent is provided in the combustion chamber 31, an example of the agent includes single base smokeless powder consisting of 98% by mass of nitrocellulose, 0.8% by mass of diphenylamine, and 1.2% by mass of potassium sulfate. Any of various gas generation agents for use in an airbag gas generator or a seatbelt pretensioner gas generator may be used. When the gas generation agent is also used, the falling ratio in the generation pressure is small since prescribed gas generated during combustion includes a gaseous component at normal temperatures unlike the case of using only the gunpowder 22. In addition, the time required for completion of combustion during combustion of the gas generation agent is extremely longer than the gunpowder 22 but the time until the completion of combustion of the gas generation agent can be changed by adjusting the dimension or size and shape of the gas generation agent, especially the shape of the surface thereof when the agent is provided in the combustion chamber 31. In this way, the generation pressure in the combustion chamber 31 may be adjusted, as appropriate, by adjusting the amount, shape, and arrangement of the gas generation agent.

Note that nozzle portions 9 formed in the nozzle main body 10 may be plural, or may be single. When multiple nozzle portions are formed, flow passages corresponding to the nozzle portions are preferably formed so that a released injection solution ML is delivered to the nozzles as equally as possible, as shown in Fig. 1(c). Furthermore, when multiple nozzle portions 9 are formed, the nozzle portions are preferably provided to be exposed at equal intervals around the center axis of the injector 1 and fixed by the nozzle holder 12. The flow passage size of the nozzle portion 9 is set to be smaller than the inner diameter of the through hole 34. In this way, the ejection pressure of the injection solution during ejection can optimally be raised.

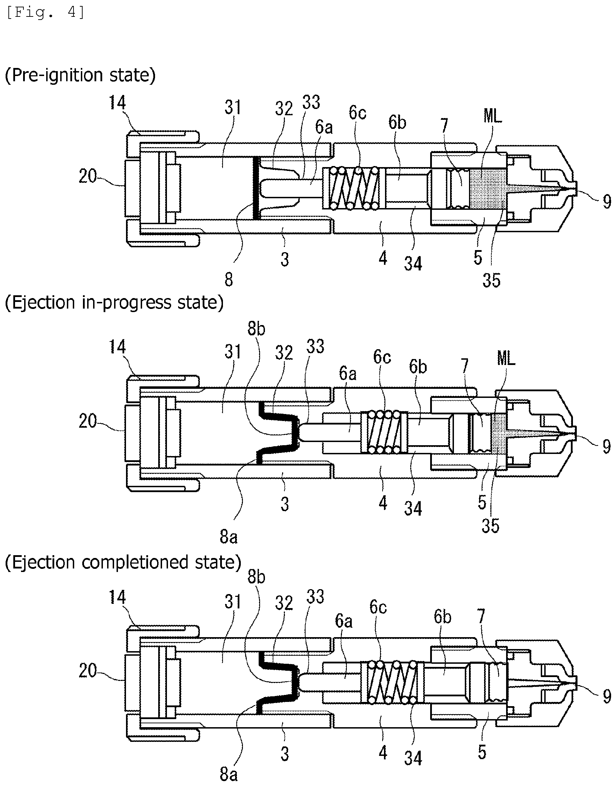

Here, the ejection state of the injection solution in the injector 1 will be described with reference to Fig. 4 along with the movement of the pressing member 8 and the piston portion 6 when the gunpowder 22 in the initiator 20 is combusted. Fig. 4 shows the state of the injector 1 before ignition in the upper stage, the state of the injector 1 in which the ejection of the injection solution by the combustion of the gunpowder in the initiator 20 is in progress in the middle stage (hereinafter referred to as an "ejection in-progress state"), and the state of the injector 1 in which the ejection of the injection solution is completed (hereinafter referred to as an "ejection completion state") in the lower stage.

As shown in Fig. 2, in the pre-combustion state, the pressing member 8 has its peripheral part 8a fixed to the fitting part 4a, and the pressing member 8 is substantially in a flat plate shape. Here, when the gunpowder 22 is combusted in the initiator 20, the combustion product thereof is diffused in the combustion chamber 31, and the pressure inside the combustion chamber 31 increases. In this way, the pressure is also applied upon the pressing member 8. As a result, as in the ejection in-progress state, the pressing member 8 plastically deforms to be depressed toward the recess 32. At the time, since the pressing member 8 has its peripheral part 8a fixed, the center part 8b plastically deforms to be displaced toward the side of the first end portion 6a of the piston portion 6. Therefore, the plastic deformation causes the center part 8b to press the first end portion 6a, which causes the piston portion 6 to slide in the through hole 34.

Here, as for the pressing of the piston portion 6 by the center part 8b of the pressing member 8, the pressure increase inside the combustion chamber 31 caused by the combustion of the gunpowder 22 is abrupt, and therefore the center part 8b abruptly presses the first end portion 6a of the piston portion 6 to cause the entire piston portion 6 to slide, while the spring part 6c of the piston portion 6 elastically deforms, in other words, compressively deforms. This indicates that ejection energy generated by the combustion of the gunpowder 22 is partly accumulated at the spring part 6c as elastic energy. As a result, substantially in the first half of the ejection process of the injection solution (from the pre-combustion state to the ejection in-progress state in Fig. 4), the injection solution ML is ejected from the nozzle portion 9 by the movement of the entire piston portion 6 (sliding in the through hole 34). Note that at the time, the pressing member 8 presses the piston portion 6 through the center part 8b.

Substantially in the following last half of the process of ejecting the injection solution (from the ejection in-progress state to the ejection completion state in Fig. 4), the elastic energy accumulated at the spring part 6c substantially in the first half of the process is released, which causes the second end portion 6b of the piston portion 6 in contact with the plunger 7 to be pushed toward the plunger 7. Note that at the time, the entire piston portion 6 may slide in the through hole 34 or may stop sliding in the through hole 34. More specifically, substantially in the last half of the process, at least the second end portion 6b is pushed toward the plunger 7, so that the injection solution ML is ejected. Therefore, in the injector 1 according to the present invention, the length of the through hole 34 is designed so that the elastic energy by the spring part 6c compressed by the combustion of the gunpowder 22 is released, and the injection solution ML is ejected. Stated differently, the length of the through hole 34 is determined so that the piston portion 6 is prevented from pressing the plunger 7 to complete the ejection of the injection solution ML while the spring part 6c compressed by the combustion of the gunpowder 22 is still in its most compressed state, and this secures a space which allows the elastic deformation of the spring part 6c to partly or entirely recover and the second end portion 6b to be pushed toward the plunger 7.

As described above, the pressing member 8 plastically deforms by pressurization generated by the combustion of the gunpowder 22, and therefore substantially in the last half of the process in which the spring part 6c recovers from the elastic deformation, the first end portion 6a of the piston portion 6 is supported by the center part 8b of the pressing member 8. Therefore, the first end portion 6a is prevented from being pushed back toward the initiator 20, so that the elastic energy released from the spring part 6c is efficiently used for pushing the second end portion 6b toward the plunger 7. As a result, the injection solution ML can be ejected smoothly.

In the injector 1 configured to allow the injection solution ML to be ejected, the entire piston portion 6 slides upon being pressed by the pressing member 8, so that the ejection energy is directly transmitted to the plunger 7 while substantially in the last half of the ejection process, in particular, the elastic energy accumulated at the spring part 6c is transmitted to the plunger 7. As a result, the combustion of the gunpowder in the initiator 20 causes the piston portion 6 to pressurize the injection solution ML through the plunger 7 for a relatively long period of time. This allows efficient ejection of the injection objective substance, and the initiator 20 as well as the injector 1 can be reduced in size. In the injector 1, the pressing member 8 is provided to separate the combustion chamber 31 and the through hole 34 as the internal spaces of the injector main body 2 and seal the combustion product generated by the initiator 20 inside the combustion chamber 31. In this way, the combustion product does not enter the through hole 34, so that any undesirable effect of the combustion product on the injection solution ML can be reduced.

### <Modification>

According to the embodiment, the pressing member 8 is made of a plate shaped metal member which plastically deforms by pressure generated by the combustion of the gunpowder 22 but alternatively, the pressing member may be displaced toward the side of the piston portion 6 so that the member does not return to the side of the initiator 20 by the pressure. For example, the pressing member 8 may be configured like a ratchet, so that displacement only in one direction is allowed. In this case, upon receiving pressure by combustion of the gunpowder 22, the pressing member 8 fixed to the injector main body 2 is partly displaced toward the side of the piston portion 6 to press the first end portion 6a.

### <Second Embodiment>

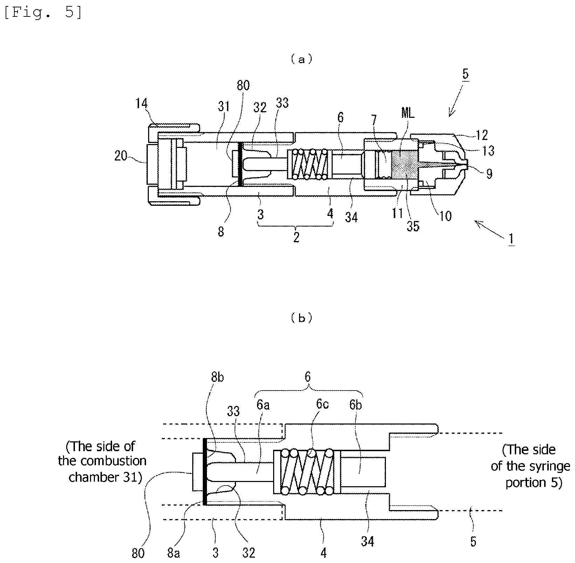

Fig. 5 shows a second embodiment of the present invention. Similarly to Fig. 1, a general structure of an injector 1 according to the embodiment is illustrated in the upper stage in Fig. 5, and similarly to Fig. 2, the positional relation between the piston portion 6 and the second housing 4 is shown in the lower stage in Fig. 5. Note that in the following description of the embodiment, the elements substantially identical to those of the first embodiment are designated by the same reference characters and a detailed description thereof will not be provided.

According to the embodiment, in the pre-combustion state in Fig. 5, a reinforcement member 80 is provided on the initiator side surface of the center part 8b of the pressing member 8, in other words on the surface opposite to the surface opposed to the first end portion 6a of the piston portion 6. The center part 8b of the pressing member 8 presses the first end portion 6a when the gunpowder 22 is combusted in the initiator 20 as described above, and therefore relatively large stress is applied on the part upon ejection of an injection solution ML. Therefore, the reinforcement member 80 is provided on the surface of the center part 8b on the side of the initiator 20, so that the pressing member 8 may be provided with sufficient strength in the thickness-wise direction of the member with respect to the stress associated with the ejection, so that the injection solution ML can be ejected smoothly and the combustion product can securely be sealed inside the combustion chamber 31.

Note that the pressing member 8 plastically deforms in response to pressure applied thereon by the combustion of the gunpowder 22 as described above, the reinforcement member 80 is preferably provided in a prescribed location of the center part 8b which does not prevent the plastic deformation. In particular, the reinforcement member 80 is preferably provided on the opposite side to the end face of the first end portion 6a through the center part 8b.

### <Other Embodiments>

Using the injector 1 according to the present invention, for example cultured cells or stem cells can be seeded to cells or scaffold tissues (scaffolds) as an injection subject in the field of human regenerative medicine in addition to the injection of the injection solution to the skin structure as described above. For example, as disclosed in Japanese Patent Application Publication No. 2008-206477, the injector 1 may be used for injection of cells which may be determined, as appropriate, by a person skilled in the art depending on the site for transplant and the purpose of recellularization, and examples of the cells include endothelial cells, endothelial progenitor cells, myeloid cells, preosteoblast cells, cartilage cells, fibroblast cells, skin cells, muscle cells, liver cells, kidney cells, intestinal tract cells, stem cells, and any other cells which may be considered for use in the field of regenerative medicine. More specifically, a solution containing the cells to be seeded as described above (a cell suspension) is stored in the storing chamber 35 and pressurized, so that the prescribed cells are injected and transplanted to the site for transplant.

Furthermore, the injector 1 according to the present invention can also be used to deliver DNA or the like to cells or scaffold tissues (scaffolds) as disclosed in Japanese Translation of PCT Application No. 2007-525192. In this case, the cells or scaffold tissues (scaffolds) are less affected by the use of the injector 1 than in delivery through a needle and therefore the use of the injector according to the present invention is more preferable.

Furthermore, the injector 1 according to the present invention may also be optimally used in directly delivering various genes, cancer suppressor cells, or lipid envelops to a target tissue or in administering an antigen gene for increasing immunity to a pathogen. Furthermore, the injector 1 may also be used optimally in the field of medical treatment of various diseases (the field as disclosed in Japanese Translation of PCT Application No. 2008-508881 and Japanese Translation of PCT Application No. 2010-503616) and the field of immunological medical treatment (the field as disclosed in Japanese Translation of PCT Application No. 2005-523679) and it is not intended to limit the applicable fields.

### [Reference Signs List]

- 1: Injector
- 2: Injector main body
- 5: Syringe portion
- 6: Piston
- 7: Plunger
- 8: Pressing member
- 9: Nozzle portion
- 10: Nozzle holder
- 11: Syringe portion main body
- 20: Initiator
- 22: Gunpowder
- 31: Combustion chamber
- 32: Recess
- 33: Through hole
- 34: Through hole
- 35: Storing chamber

## Claims

1. A needleless injector for injecting an injection objective substance to an injection target area without using an injection needle, the needleless injector comprising:
an injector main body having a through hole formed in an axial direction thereof;
a piston portion provided slidably in the through hole;
a syringe portion provided on a tip end side of the injector main body, the syringe portion including a storing chamber capable of storing the injection objective substance, a plunger to pressurize the injection objective substance in the storing chamber as the piston portion slides; a nozzle portion that includes a flow passage for allowing the injection objective substance in the storing chamber pressurized by the plunger to be flown therethrough and ejects the injection objective substance from an ejection outlet formed at a tip end of the flow passage;
an igniting device provided at a base end side of the injector main body to burn gunpowder, the igniting device supplying ejection energy by combustion of the gunpowder in the igniting device; and
a pressing member provided between the piston portion and the igniting device, the pressing member being formed so as to have a part displaceable toward the piston portion while the part being kept from returning to the igniting device side upon receiving the ejection energy from the igniting device, and pressing the piston portion toward the plunger due to displacement of the part, wherein
the piston portion has an elastically deformable part which elastically deforms in a sliding direction of the piston portion upon receiving pressing force from the pressing member, and an output portion provided in contact with the plunger and connected to the elastically deformable part, and in the sliding process of the piston portion, while the piston portion is pressed by the pressing member, the elastically deformable part is partly or entirely recovered from the elastic deformation from a most compressed state by the pressing force from the pressing member so as to push the output portion toward the plunger.

2. The needleless injector according to claim 1, wherein the pressing member plastically deform toward the piston portion upon receiving the ejection energy from the igniting device, so that a part of the pressing member is displaced toward the piston portion and presses the piston portion.

3. The needleless injector according to claim 1 or 2, wherein
the pressing member is formed to separate the space in the injector main body into a first space in which the igniting device is provided and a second space in which the piston portion is provided and to seal a combustion product generated by the igniting device in the first space.

4. The needleless injector according to any one of claims 1 to 3, wherein
the pressing member is made of a metal plate member or a metal film member.

5. The needleless injector according to claim 4, wherein a reinforcement member which reinforces the pressing member in a thickness-wise direction is provided in a prescribed location of the pressing member in contact with the piston portion.
